# FASCICULE DE BREVET EUROPEEN

(11) **EP 2 912 014 B1**
(45) Date de publication et mention de la délivrance du brevet: **14.12.2016**
(21) Numéro de dépôt: 13777062.4
(22) Date de dépôt: 16.10.2013
(51) Int. Cl.: C07C 323/36, C08G 18/64, C09J 175/02, C08G 18/38, C08G 18/76, C08G 18/80, C08G 18/32

(54) **POLYAMINE POLYAROMATIQUE SOUFREE UTILISABLE POUR LA SYNTHESE DE POLYUREE**
SULPHURISIERTES POLYAROMATISCHES POLYAMIN ZUR SYNTHESE VON POLYHARNSTOFF
SULPHURETTED POLYAROMATIC POLYAMINE THAT CAN BE USED IN THE SYNTHESIS OF POLYUREA

(30) Priorité: 24.10.2012 FR 1260101
(43) Date de publication de la demande: 02.09.2015
(73) Titulaire: Compagnie Générale des Etablissements Michelin, 63000 Clermont-Ferrand (FR); MICHELIN Recherche et Technique S.A., 1763 Granges-Paccot (CH)
(72) Inventeur: FEDURCO, Milan, 63040 Clermont-Ferrand Cedex 9 (FR); RIBEZZO, Marco, 63040 Clermont-Ferrand Cedex 9 (FR)
(74) Mandataire: Ribière, Joel
(86) Numéro de dépôt international: PCT/EP2013/071619
(87) Numéro de publication internationale: WO 2014/063968

(56) Documents cités:
- US-A1- 2010 021 676

## Description

### I. DOMAINE DE L'INVENTION

La présente invention est relative aux monomères ou prépolymères utilisables pour la synthèse de polyurées (polymères à unités urée) destinées notamment à des systèmes adhésifs pour collage de métal ou de verre à du caoutchouc, en particulier dans des composites métal / caoutchouc destinés aux articles en caoutchouc tels que des pneumatiques.

Elle est plus particulièrement relative aux monomères ou prépolymères ci-dessus du type polyamines polyaromatiques soufrées.

### II. ETAT DE LA TECHNIQUE

Les composites métal / caoutchouc, en particulier pour pneumatiques, sont bien connus. Ils sont généralement constitués d'une matrice en caoutchouc insaturé généralement diénique, réticulable au soufre, comportant des éléments de renforcement (ou « renforts ») métalliques tels que des fils, films ou câbles en acier au carbone.

Soumis à des contraintes très importantes lors du roulage des pneumatiques, notamment à des compressions, flexions ou variations de courbure répétées, ces composites doivent de manière connue satisfaire à un grand nombre de critères techniques, parfois contradictoires, tels qu'uniformité, flexibilité, endurance en flexion et en compression, résistance à la traction, à l'usure et à la corrosion, et maintenir ces performances à un niveau très élevé aussi longtemps que possible.

On comprend aisément que l'interphase adhésive entre caoutchouc et métal joue un rôle prépondérant dans la pérennité de ces performances. Le procédé traditionnel pour relier les compositions de caoutchouc à de l'acier au carbone consiste à revêtir la surface de l'acier avec du laiton (alliage cuivre-zinc), la liaison entre l'acier et la matrice de caoutchouc étant assurée par sulfuration du laiton lors de la vulcanisation ou cuisson du caoutchouc. Pour améliorer l'adhésion, on utilise en outre généralement, dans ces compositions de caoutchouc, des sels organiques ou des complexes de métal tels que cobalt, en tant qu'additifs promoteurs d'adhésion. US2010/021676A1 décrit une colle à base de polyurée pour faire adhérer deux parties l'une à l'autre, comme des compositions de caoutchouc ou du métal et du caoutchouc. Pour la préparation de cette colle à base de polyurée on mélange un catalyseur de trimérisation, un polymère fonctionnel comportant des groupes amines et ETHACURE TM 300 (allongeur de c haine qui est la 3,5-diméthylthio-2,4- (et -2,6-) toluène diamine) et un trimère dérivé de l'hexamethylène diisocyanates.

Or, on sait que l'adhésion entre l'acier au carbone et la matrice de caoutchouc est susceptible de s'affaiblir au cours du temps, du fait de l'évolution progressive des sulfures formés sous l'effet des différentes sollicitations rencontrées, notamment mécaniques et/ou thermiques, le processus de dégradation ci-dessus pouvant être accéléré en présence d'humidité.

D'autre part, l'utilisation de sels de cobalt rend les compositions de caoutchouc plus sensibles à l'oxydation et au vieillissement, et en augmente significativement le coût, sans compter qu'il est souhaitable de supprimer à terme l'emploi de tels sels de cobalt dans les compositions de caoutchouc, en raison de l'évolution récente de la réglementation européenne sur ce type de sels métalliques.

Pour toutes les raisons exposées ci-dessus, les fabricants de composites métal / caoutchouc, en particulier les manufacturiers de pneumatiques, sont à la recherche de solutions adhésives nouvelles pour faire coller les renforts métalliques aux compositions de caoutchouc, tout en palliant, au moins en partie, les inconvénients précités.

### III. BREVE DESCRIPTION DE L'INVENTION

Or, au cours de leurs recherches, les Demanderesses ont trouvé un composé polyamine nouveau, plus précisément une polyamine polyaromatique soufrée spécifique qui est utilisable, comme monomère ou (lorsque n est différent de zéro dans la formule Ici-dessous) comme prépolymère, pour la synthèse d'une polyurée qui répond à un tel objectif.

Selon l'invention, ladite polyamine polyaromatique soufrée répond à la formule (I) :

H₂N-Ar₁-NH-CH₂-CH(OH)-(CH₂)ₘ-O-Z₁-O-(CH₂)ₘ-CH(OH)-CH₂-[X]ₙ-HN-Ar₂-NH₂

dans laquelle X représente l'enchaînement :

-HN-Ar₃-NH-(CH₂)-CH(OH)-(CH₂)ₘ-O-Z₂-O-(CH₂)ₘ-CH(OH)-(CH₂)-

et dans laquelle:
- le symbole « n » représente un nombre entier égal à zéro ou différent de zéro ;
- les symboles « m », identiques ou différents, représentent un nombre entier compris dans un domaine de 1 à 10 ;
- les symboles Z₁ et Z₂, identiques ou différents, représentent un groupement de liaison divalent comportant de 1 à 30 atomes de carbone ;
- Ar₁, Ar₂ et Ar₃, identiques ou différents, représentent un groupe phénylène, un au moins de ces groupes phénylène étant porteur de un, deux, trois ou quatre groupements de formule -Sₓ-R dans laquelle « x » est un entier de 1 à 8 et R représente l'hydrogène ou un groupe hydrocarboné, pouvant comporter un hétéroatome, comportant de 1 à 10 atomes de carbone.

Grâce à cette polyamine conforme à l'invention, il est possible de préparer une polyurée qui, utilisée comme primaire d'adhésion par exemple sur des renforts métalliques, donne comme avantage majeur à de tels renforts celui de pouvoir ensuite être collés à des matrices de caoutchouc insaturé en utilisant de simples colles textiles telles que des colles « RFL » (résorcinol-formaldéhyde-latex) ou autres compositions adhésives équivalentes, ou encore directement (c'est-à-dire sans emploi de telles colles) à ces matrices de caoutchouc insaturé lorsque ces dernières contiennent des élastomères insaturés fonctionnalisés appropriés tels que des élastomères époxydés.

L'invention concerne également l'utilisation d'un composé polyamine conforme à l'invention pour la fabrication d'une polyurée, ainsi que toute polyurée issue d'au moins un composé polyamine conforme à l'invention.

L'invention concerne également tout procédé de synthèse d'une polyurée par polycondensation d'au moins un composé conforme à l'invention avec un composé polyisocyanate.

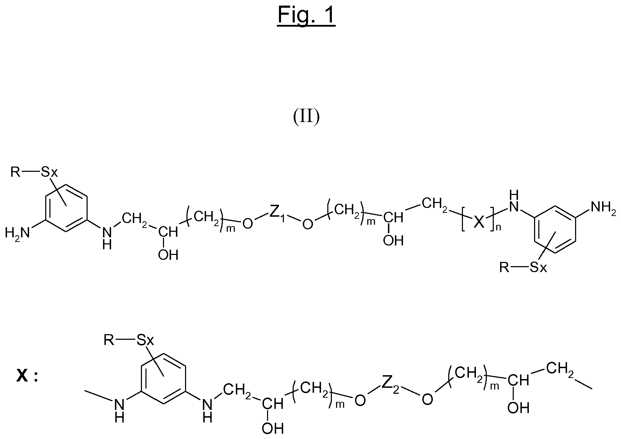

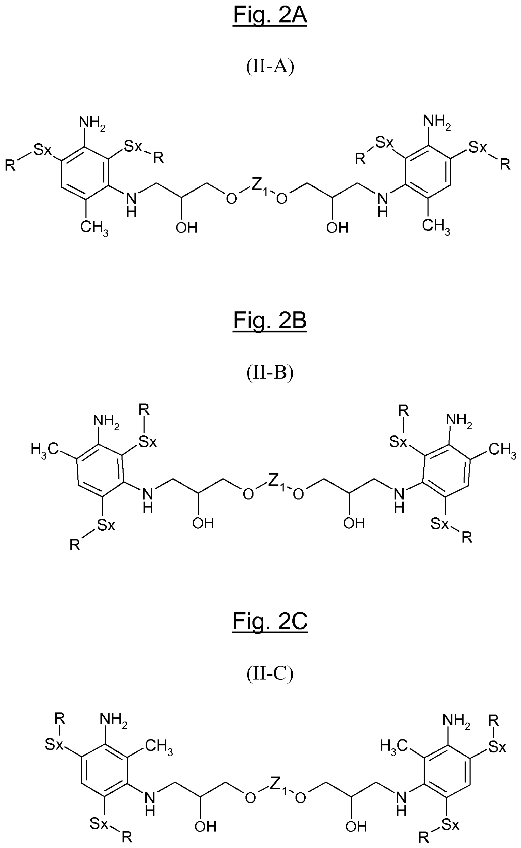

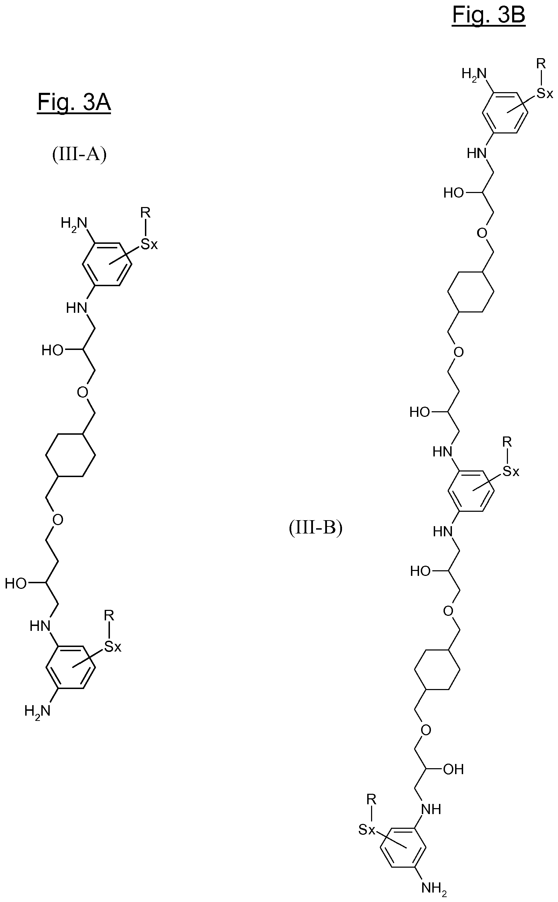

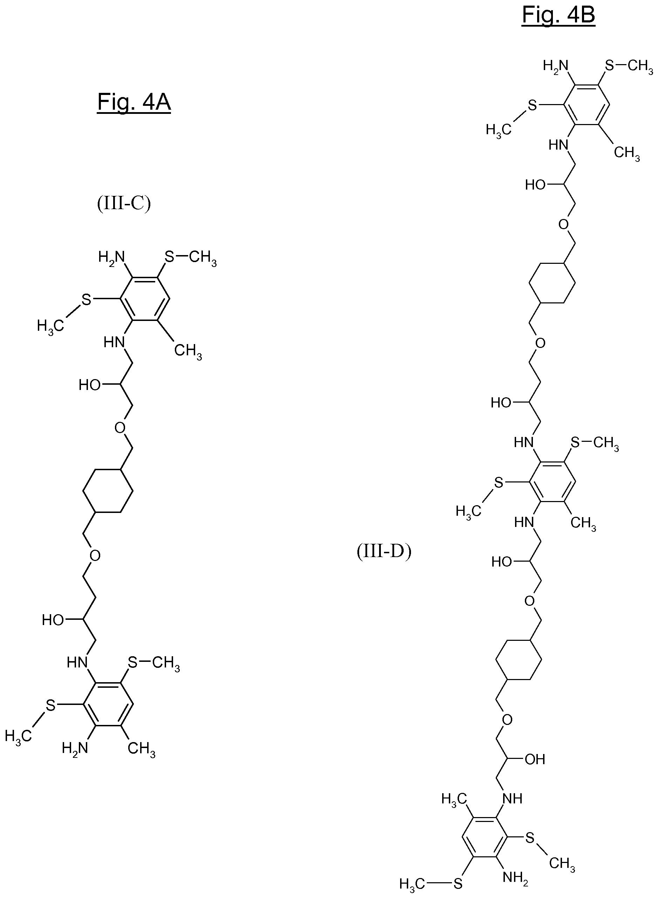

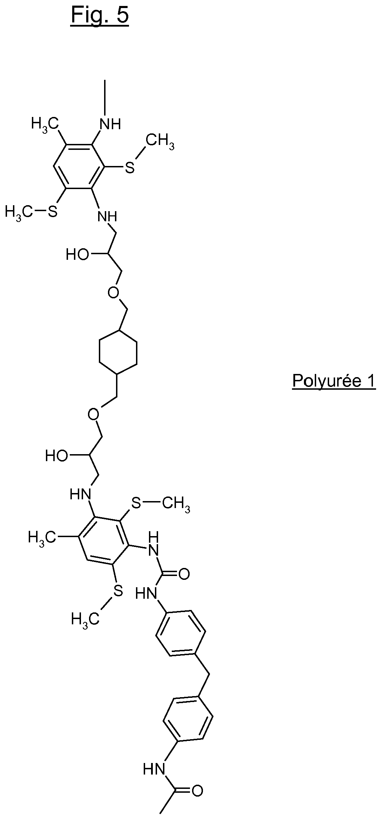

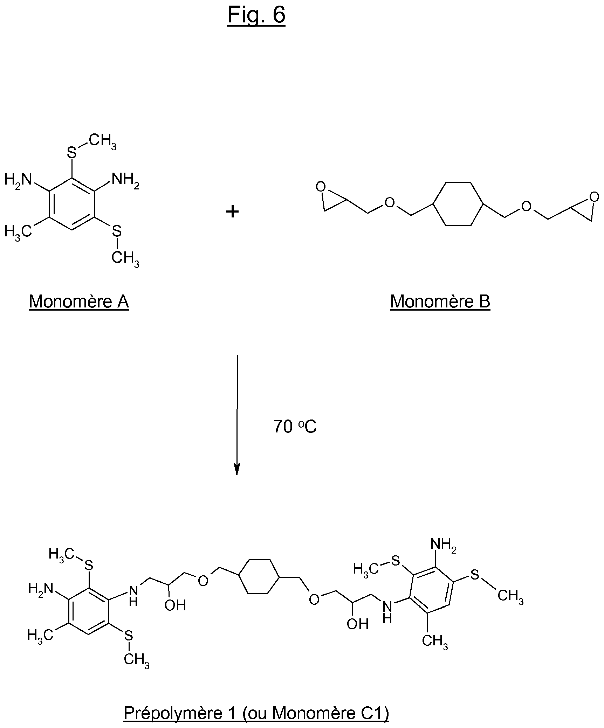

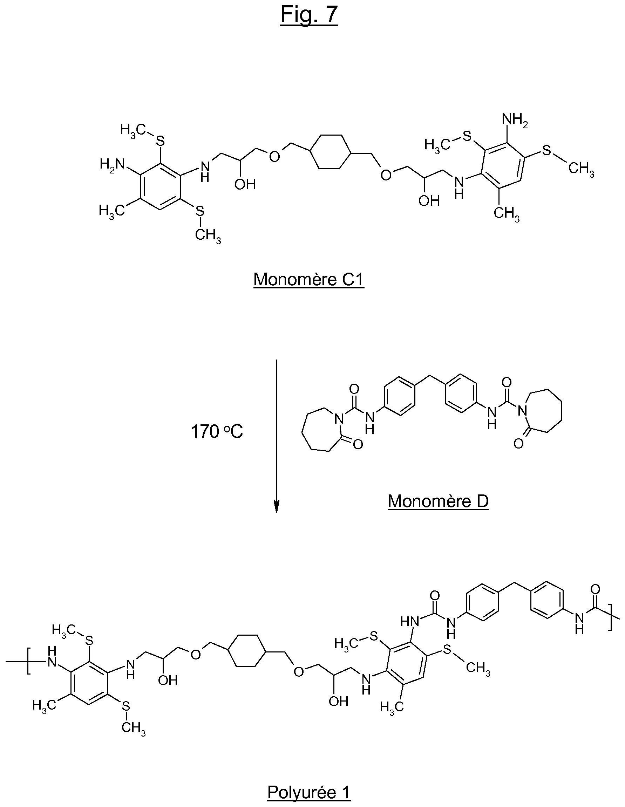

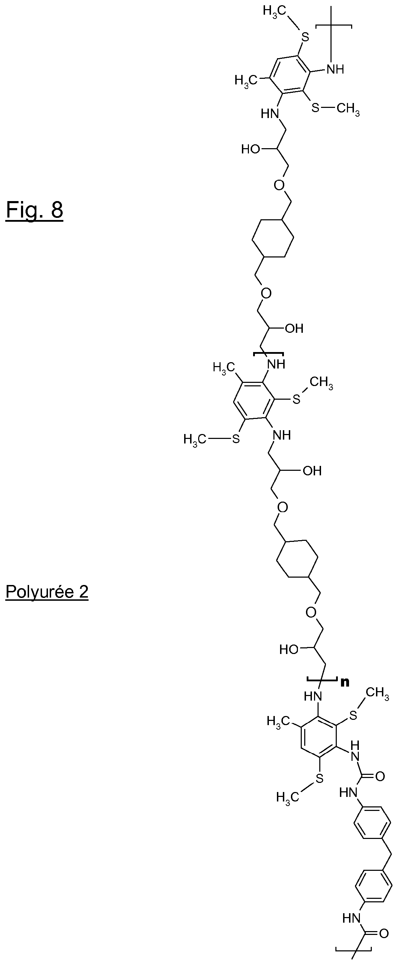

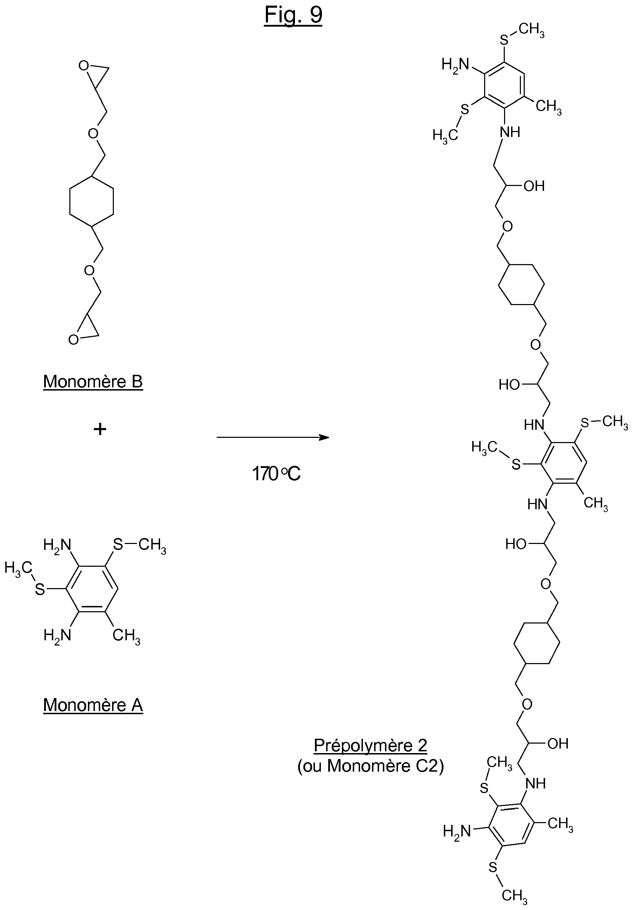

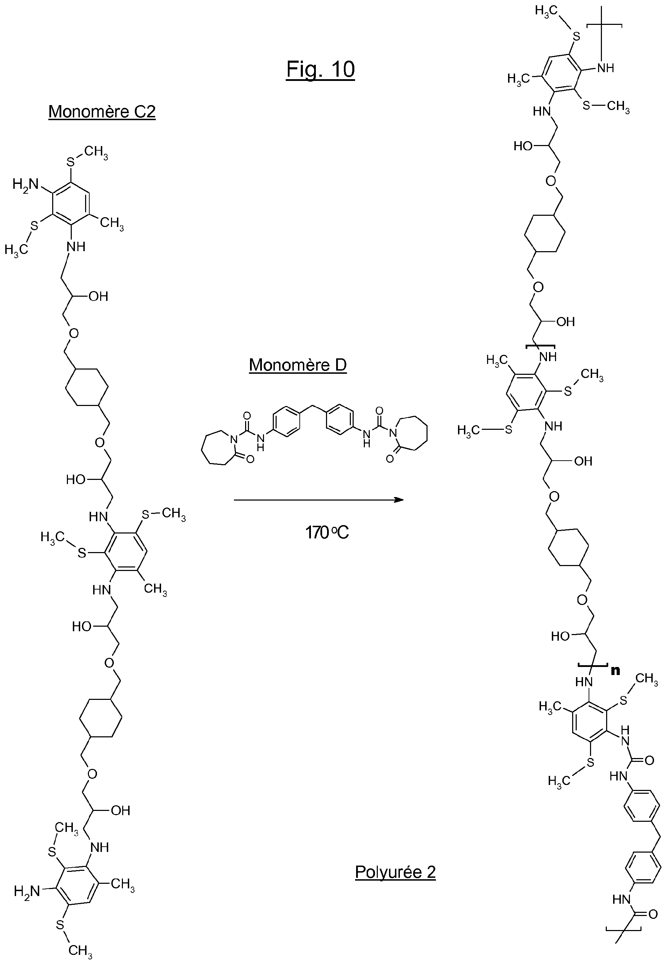

L'invention ainsi que ses avantages seront aisément compris à la lumière de la description détaillée et des exemples de réalisation qui suivent, ainsi que des figures relatives à ces exemples qui représentent ou schématisent :
- un exemple de polyamine polyaromatique soufrée dont le motif de formule générale (I) ci-dessus répond à la formule particulière (II) dans laquelle, sur chaque groupe phénylène, les groupes amino (NH et/ou NH₂) sont en position méta l'un par rapport à l'autre (Fig. 1) ;
- trois exemples de polyamines polyaromatiques soufrées dont le motif de formule particulière (II) répond à la formule plus particulière (II-A), (II-B) ou (II-C) dans lesquelles « m » est égal à 1 pour chaque groupe (CH₂)ₘ et « n » est égal à zéro (respectivement Fig. 2A, 2B et 2C) ;
- deux exemples de polyamines polyaromatiques soufrées dont le motif de formule particulière (II) répond à la formule plus particulière (III-A) ou (III-B) dans lesquelles « m » est égal à 1 pour chaque groupe (CH₂)ₘ, Z₁ et le cas échéant Z₂ représentent le 1,4-diméthylène cyclohexane, et « n » est égal à 0 (III-A) ou à 1 (III-B) (respectivement Fig. 3A et 3B) ;
- deux exemples de polyamines polyaromatiques soufrées dont les motifs de formules particulières (III-A) ou (III-B) répondent respectivement aux formules plus particulières (III-C) et (III-D) dans lesquelles, en outre, chaque groupement phénylènediamino répond à la formule particulière (III-a) (respectivement Fig. 4A et 4B) ;
- un exemple de séquence (unité structurelle récurrente) d'un polymère (Polyurée 1) issu de la réaction d'un diisocyanate MDI (ou précurseur de MDI) et d'une polyamine conforme à l'invention (Prépolymère 1) (Fig. 5) ;
- un schéma de synthèse possible, à partir de deux Monomères A et B, d'une polyamine (Prépolymère 1, aussi appelé Monomère C1) conforme à l'invention servant à la préparation du polymère Polyurée 1 (Fig. 6) ;
- un schéma de synthèse possible du polymère Polyurée 1 à partir de la polyamine (Monomère C1) conforme à l'invention précédente et d'un diisocyanate (MDI) bloqué (dit Monomère D) (Fig. 7) ;
- un exemple de séquence (unité structurelle récurrente) d'une autre polyurée (Polyurée 2), issue de la réaction d'un diisocyanate MDI (ou précurseur de MDI) et d'une polyamine conforme à l'invention (Prépolymère 2) (Fig. 8) ;
- un schéma de synthèse possible, à partir des deux Monomères A et B, d'une polyamine conforme à l'invention (Prépolymère 2 ou Monomère C2) servant à la préparation du polymère Polyurée 2 (Fig. 9) ;
- un schéma de synthèse possible du polymère Polyurée 2 à partir de la polyamine (Monomère C2) conforme à l'invention précédente et d'un diisocyanate (MDI) bloqué (Monomère D) (Fig. 10).

### IV. DESCRIPTION DETAILLEE DE L'INVENTION

On rappellera tout d'abord ici qu'une polyurée est un polymère (par définition tout homopolymère ou copolymère, notamment copolymère à blocs) comportant une pluralité de liaisons urée (-NH-CO-NH-) résultant de manière connue de la réaction d'addition d'une polyamine ayant au moins deux fonctions amine primaire sur un polyisocyanate (composé porteur d'au moins deux fonctions isocyanate -NCO), notamment sur un diisocyanate dans le cas d'une polyurée du type linéaire.

Le composé polyamine polyaromatique de l'invention a donc pour caractéristique essentielle de répondre à la formule (I) ci-après :

(I) H₂N-Ar₁-NH-CH₂-CH(OH)-(CH₂)ₘ-O-Z₁-O-(CH₂)ₘ-CH(OH)-CH₂-[X]ₙ-HN-Ar₂-NH₂ dans laquelle:

- X représente l'enchaînement :

   -HN-Ar₃-NH-(CH₂)-CH(OH)-(CH₂)ₘ-O-Z₂-O-(CH₂)ₘ-CH(OH)-(CH₂)-
- « n » est un nombre entier égal à zéro ou différent de zéro ;
- les symboles « m », identiques ou différents, représentent un nombre entier compris dans un domaine de 1 à 10, de préférence de 1 à 5 ;
- les symboles Z₁ et Z₂, identiques ou différents, représentent un groupement de liaison divalent comportant de 1 à 30 atomes de carbone ;
- Ar₁, Ar₂ et Ar₃, identiques ou différents, représentent un groupe phénylène, un au moins de ces groupes phénylène étant porteur de un, deux ou trois groupements de formule -Sₓ-R dans laquelle « x » est un entier de 1 à 8 et R représente l'hydrogène ou un groupe hydrocarboné, pouvant comporter un hétéroatome, comportant de 1 à 10 atomes de carbone.
« x » est un entier de 1 à 8, de préférence de 1 à 4, plus préférentiellement égal à 1 ou 2, plus particulièrement égal à 1.

R représente l'hydrogène ou un groupe hydrocarboné comportant de 1 à 10 atomes de carbone, substitué ou non substitué, pouvant comporter un hétéroatome tel que S, O ou N. De préférence, R est un groupe aliphatique, plus préférentiellement un groupe alkyle. Plus préférentiellement encore, R est un alkyle ayant de 1 à 5 atomes de carbone, cet alkyle pouvant être substitué ou non substitué ; plus particulièrement, R représente un méthyle ou un éthyle, encore plus particulièrement un méthyle.

Dans les cas particuliers où, dans le groupement -Sₓ-R du motif (I) ci-dessus, R représente l'hydrogène, l'homme du métier comprendra que, lors de la synthèse de la polyamine polyaromatique de l'invention, par réaction d'une diamine de départ (par exemple monomère A ci-après) sur un composé diépoxy (par exemple monomère B ci-après), le groupe thiol -SH pourra être protégé contre toute réaction parasite avec ce composé diépoxy, de manière connue, par un groupement bloquant approprié et connu de l'homme de l'art, par exemple tel que représenté dans les formules ci-après :

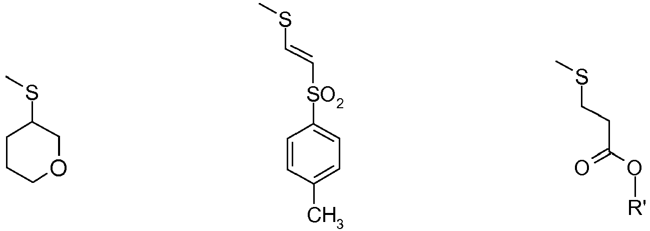

Selon un mode préférentiel de l'invention, chacun des groupes Ar₁, Ar₂ et Ar₃, identiques ou différents, est porteur de un, deux, trois ou quatre groupements de formule -Sₓ-R, en particulier de deux groupes -SR (x égal à 1) dans lesquels R est un alkyle ayant de 1 à 5 atomes de carbone, R étant plus particulièrement un méthyle ou un éthyle, encore plus particulièrement un méthyle.

Selon un mode de réalisation plus préférentiel, Ar₁, Ar₂ et Ar₃, identiques ou différents, sont chacun porteurs de deux groupes -Sₓ-R, en particulier de deux groupes -SR (x égal à 1) dans lesquels R est un alkyle ayant de 1 à 5 atomes de carbone, R étant plus particulièrement un méthyle ou un éthyle, encore plus particulièrement un méthyle. Plus préférentiellement encore, dans un tel cas, les deux groupes (-SR), en particulier (-SCH₃) sont en position méta (ou position -1,3) l'un par rapport à l'autre sur chaque groupe phénylène Ar.

Les nombres « m » peuvent être identiques ou différents d'un groupe (CH₂)ₘ à l'autre ; préférentiellement, chaque nombre « m » est égal à 1 ou 2, plus préférentiellement égal à 1.

Les symboles Z₁ et Z₂, identiques ou différents, représentent un groupement de liaison divalent, de préférence hydrocarboné mais pouvant aussi comporter un hétéroatome tel que S, O ou N ; ils comportent de 1 à 30, de préférence de 2 à 20 atomes de carbone, plus préférentiellement de 2 à 10 atomes de carbone.

Plus préférentiellement, Z₁ et Z₂ représentent un groupement aliphatique comportant de 2 à 20 atomes de carbone ou cycloaliphatique comportant de 3 à 20 atomes de carbone, encore plus préférentiellement un groupement aliphatique ou cycloaliphatique comportant de 3 à 10 atomes de carbone. Parmi ces groupements plus préférentiels, on citera en particulier le 1,4-diméthylène cyclohexane, de formule :

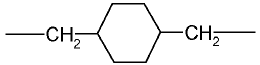

Selon un autre mode de réalisation préférentiel, « n » est égal à zéro. Dans un tel cas, le composé conforme à l'invention de formule (I) a donc la formule simplifiée qui suit :

H₂N-Ar₁-NH-CH₂-CH(OH)-(CH₂)ₘ-O-Z₁-O-(CH₂)ₘ-CH(OH)-CH₂-HN-Ar₂-NH₂.

En particulier, lorsque « n » est égal à zéro et « m » est égal à 1 pour chaque groupe (CH₂)ₘ, le composé selon l'invention de formule (I) a alors pour formule plus simplifiée :

H₂N-Ar₁-NH-CH₂-CH(OH)-CH₂-O-Z₁-O-CH₂-CH(OH)-CH₂-HN-Ar₂-NH₂.

Selon un autre mode de réalisation préférentiel, « n » est égal à 1 ou supérieur à 1 ; lorsque « n » est supérieur à 1, il est notamment compris dans un domaine de 2 à 20, plus préférentiellement de 2 à 10, en particulier de 2 à 5.

Les groupements phénylènediamino, monovalents ou divalents selon les cas (H₂N-Ar₁-NH-, -HN-Ar₂-NH₂- et -HN-Ar₃-NH-) peuvent être identiques ou différents.

Sur chaque groupe phénylène Ar, les deux groupes amino (selon les cas, soit 2 groupes NH, soit un groupe NH et un groupe NH₂) peuvent être en position méta, ortho ou para, l'un par rapport à l'autre.

Selon un mode de réalisation préférentiel, sur chaque groupe phénylène Ar, ces deux groupes amino sont en position méta (ou position -1,3) l'un par rapport à l'autre. En d'autres termes, dans un tel cas, le motif de formule générale (I) a donc pour formule développée (II) celle qui a été représentée à la figure 1 annexée.

Dans un tel mode préférentiel, les enchaînements phénylènediamino monovalents H₂N-Ar₁-NH- et -HN-Ar₂-NH₂ , identiques ou différents, répondent en particulier à l'une des formules (III-a), (III-b) ou (III-c) qui suivent :

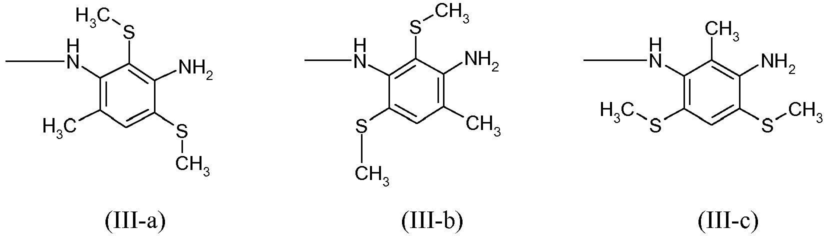

Dans un autre mode préférentiel applicable lorsque n est différent de zéro, ce mode étant plus préférentiellement combiné avec le mode préférentiel qui précède, les enchaînements phénylènediamino divalents -HN-Ar₃-NH-, identiques ou différents, répondent quant à eux à l'une des formules (III-d) ou (III-e) qui suivent :

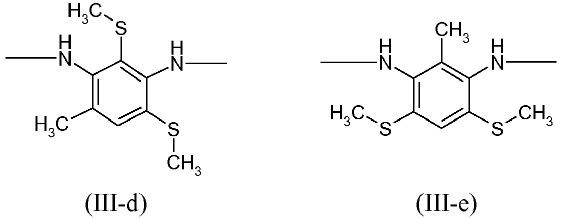

Ainsi, toujours dans le cas où les deux groupes amino sont en position méta l'un par rapport à l'autre sur chaque groupe phénylène, qu'ils répondent d'une part à l'une des formules particulières (III-a), (III-b), (III-c), (III-d) ou (III-e) ci-dessus, et que d'autre part, selon un mode de réalisation encore plus préférentiel, « m » est égal à 1 pour chaque groupe (CH₂)ₘ et que « n » est égal à 0, alors la formule (II) précédente a pour formules plus particulières les formules développées (II-A), (II-B) et (II-C) telles que représentées respectivement aux figures 2A, 2B et 2C annexées.

Toujours dans le cas où les deux groupes amino sont en position méta l'un par rapport à l'autre sur chaque groupe phénylène, et que, selon un mode de réalisation plus préférentiel, « m » est égal à 1 pour chaque groupe (CH₂)ₘ et que « n » est égal à 0, Z₁ étant par exemple le 1,4-diméthylène cyclohexane, alors la formule (II) précédente a pour formule plus particulière la formule développée (III-A) telle que représentée à la figure 3A annexée.

Toujours dans le cas où les deux groupes amino sont en position méta l'un par rapport à l'autre sur chaque groupe phénylène, et que, selon un autre mode de réalisation plus préférentiel, « m » est égal à 1 pour chaque groupe (CH₂), « n » est égal à 1, Z₁ et Z₂ étant par exemple le 1,4-diméthylène cyclohexane, alors la formule (II) précédente a pour formule plus particulière la formule développée (III-B) telle qu'elle est représentée à la figure 3B annexée.

La diamine polyaromatique soufrée conforme à l'invention précédemment décrite est utilisable préférentiellement pour la synthèse d'une polyurée du type linéaire, donc résultant essentiellement de l'addition de cette diamine et d'un diisocyanate. Le diisocyanate utilisable peut être aromatique ou aliphatique ; il peut s'agir d'un monomère, d'un prépolymère ou quasi-prépolymère, voir même d'un polymère.

Selon un mode de réalisation préférentiel, le diisocyanate est choisi dans le groupe constitué par les composés aromatiques suivants : diphénylméthane diisocyanate (en abrégé « MDI »), toluène diisocyanate (« TDI »), naphtalène diisocyanate (« NDI »), 3,3'-bitoluène diisocyanate (« TODI »), paraphénylène diisocyanate (« PPDI »), leurs différents isomères, et les mélanges de ces composés et/ou isomères.

Plus préférentiellement, on utilise un MDI ou un TDI, plus préférentiellement encore un MDI. Tous les isomères du MDI (notamment 2,2'-MDI, 2,4'-MDI, 4,4'-MDI) et leurs mélanges sont utilisables, ainsi que des MDI dits polymériques (ou « PMDI ») comportant des oligomères de formule suivante (avec p égal ou supérieur à 1) :

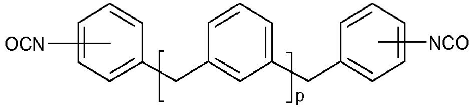

Sont également utilisables des composés diisocyanates du type aliphatiques, tels que par exemple 1,4-tétraméthylène diisocyanate, 1,6-hexane-diisocyanate (« HDI »), 1,4-bis(isocyanatométhyl)-cyclohexane, 1,3-bis(isocyanatométhyl)-cyclohexane, 1,3-bis(isocyanatométhyl)benzène, 1,4-bis(isocyanatométhyl)benzène, isophorone diisocyanate (« IPDI »), bis(4-iso-cyanatocyclohexyl)méthane diisocyanate (« H12MDI »), 4,4'-dicyclohexylméthane diisocyanate (« H13MDI »).

Selon un mode de réalisation particulièrement préférentiel, le diisocyanate utilisé est le 4,4'-MDI (4,4'-diphénylméthane diisocyanate) ayant pour formule : 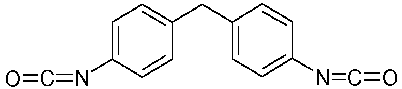 ou, si plusieurs diisocyanates sont utilisés, constitue le diisocyanate majoritaire en poids, représentant préférentiellement dans le dernier cas plus de 50% du poids total des composés diisocyanates.

On pourra utiliser avantageusement un 4,4'-MDI bloqué caprolactame (par exemple le produit sous forme solide « Grilbond » IL-6 de la société EMS), de formule :

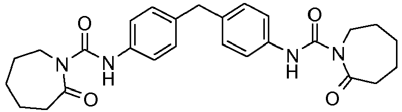

L'utilisation du composé de l'invention n'étant pas limitée à la synthèse d'une polyurée du type linéaire (cas d'un diisocyanate), on pourrait également utiliser, notamment dans le but d'augmenter la Tg du polymère par formation d'un réseau tridimensionnel, un composé triisocyanate tel que par exemple un trimère de MDI à noyau triazine de formule :

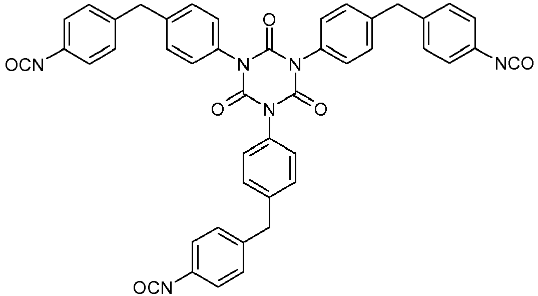

Selon un mode préférentiel de l'invention, dans le polymère polyurée issu de la polyamine de l'invention, les unités structurelles polyamines polyaromatiques soufrées dérivées de ladite polyamine de l'invention et les unités structurelles de base à unités urée (-NH-CO-NH-) sont reliées entre elles selon un motif répondant à la formule (IV) ci-dessous :

-HN-Ar₁-NH-Y-[X]ₙ-HN-Ar₂-NH-CO-NH-Z₃-NH-CO-

dans laquelle :
- Ar₁, Ar₂, X, « n », « m » et Z₁ ont les significations données précédemment ;
- Y représente l'enchaînement :

   -CH₂-CH(OH)-(CH₂)ₘ-O-Z₁-O-(CH₂)ₘ-CH(OH)-CH₂-
- et Z₃ représente un groupement de liaison divalent aliphatique, cycloaliphatique ou aromatique, le groupement aliphatique comportant de préférence 1 à 30 (plus préférentiellement 1 à 20) atomes de carbone, le groupement cycloaliphatique de préférence de 3 à 30 (plus préférentiellement 3 à 20) atomes de carbone, le groupement aromatique de 6 à 30 (plus préférentiellement 6 à 20) atomes de carbone.

Les figures 5 à 10 annexées représentent des exemples préférentiels de polyurées issues de polyamines conformes à l'invention ainsi que divers schémas de synthèse possibles de ces polyurées à partir de ces polyamines conformes à l'invention.

Tout d'abord, la figure 5 annexée représente un exemple d'enchaînement d'un polymère (ci-après « Polyurée 1 »), issu de la réaction d'un monomère MDI et d'une polyamine de départ (ci-après « Prépolymère 1 ») conforme à l'invention ayant pour formule générale :

H₂N-Ar₁-NH-CH₂-CH(OH)-(CH₂)ₘ-O-Z₁-O-(CH₂)ₘ-CH(OH)-CH₂-HN-Ar₂-NH₂,

dans laquelle Ar₁, Ar₂ et Z₁ ont les définitions générales données précédemment pour les motifs de formule (I), et plus particulièrement les caractéristiques correspondant au motif final de formule (III-A), à savoir que les deux groupes amino (H₂N- et -NH) sont en position méta l'un par rapport à l'autre sur chaque groupe phénylène (Ar₁ et Ar₂), « m » est égal à 1 pour chaque groupe (CH₂)ₘ, « n » est égal à 0 et Z₁ est le 1,4-diméthylène cyclohexane.

Les figures 6 et 7 qui suivent illustrent des exemples de procédés utilisables pour la synthèse du Prépolymère 1 et de la Polyurée 1, respectivement, procédés qui seront décrits en détail ultérieurement.

La figure 8 annexée représente un autre exemple d'enchaînement d'un polymère (« Polyurée 2 ») issu de la réaction d'un monomère MDI et d'une autre polyamine de départ (ci-après « Prépolymère 2 ») selon l'invention ayant pour formule générale :

H₂N-Ar₁-NH-(CH₂)-CH(OH)-(CH₂)ₘ-O-Z₁-O-(CH₂)ₘ-CH(OH)-(CH₂)-[X]ₙ-HN-Ar₂-NH₂

avec X répondant à la formule:

-HN-Ar₃-NH-(CH₂)-CH(OH)-(CH₂)-O-Z₂-O-(CH₂)-CH(OH)-(CH₂)-

dans laquelle Ar₁, Ar₂, Ar₃, Z₁ et Z₂ ont les définitions générales données précédemment pour les motifs de formule (I), et plus particulièrement les caractéristiques correspondant au motif final de formule (III-B), à savoir que les deux groupes amino sont en position méta l'un par rapport à l'autre sur chaque groupe phénylène (Ar₁, Ar₂ et Ar₃), «m» est égal à 1 pour chaque groupe (CH₂)ₘ, «n» est égal à 1 et Z₁ et Z₂ représentent le 1,4-diméthylène cyclohexane.

Les figures 9 et 10 qui suivent illustrent des exemples de procédés utilisables pour la synthèse du Prépolymère 2 et de la Polyurée 2, respectivement, procédés qui seront décrits en détail ultérieurement.

La polyurée synthétisable à partir d'un composé polyamine conforme à l'invention présente une température de transition vitreuse Tg, mesurée par DSC (*Differential Scanning Calorimetry*), par exemple selon ASTM D3418, qui est de préférence supérieure à 50°C, plus préférentiellement supérieure à 100°C, en particulier comprise entre 130°C et 250°C.

Comme indiqué précédemment, cette polyurée synthétisable à partir de la polyamine conforme à l'invention est avantageusement utilisable comme revêtement hydrophobe sur tout type de substrat, notamment en métal ou en verre, ou encore comme primaire d'adhésion sur tout type de renfort métallique, tel que par exemple un fil, un film, une plaque ou un câble en acier au carbone, laitonné ou non laitonné, destiné en particulier à renforcer une matrice de caoutchouc insaturé tel que du caoutchouc naturel.

### 5. EXEMPLES DE REALISATION DE L'INVENTION

Dans la présente demande, sauf indication expresse différente, tous les pourcentages (%) indiqués sont des pourcentages en masse.

Les essais qui suivent décrivent la synthèse des Prépolymères 1 et 2 conformes à l'invention notés aussi Monomères C1 et C2, à partir des Monomère A et Monomère B, puis celles de Polyurées (1, 2 et 3) à partir de ces Prépolymères 1 et 2 (Monomères C1 et C2) et d'un Monomère D (MDI).

Au cours de ces essais, divers tests d'adhésion sont conduits, sur différents substrats (verre ou métal) pour illustrer l'excellente performance adhésive des polyurées issues des polyamines conformes à l'invention.

### 5.1. Essai 1 - Préparation des Prépolymères 1 et 2 selon l'invention

Les Prépolymères 1 et 2 (aussi dénommés Monomères C1 et C2), dont les formules respectives sont données aux figures 6 et 9, ont été préparés à partir de deux Monomères A et B, selon les modes opératoires schématisés aux figures 6 et 9, comme expliqué en détail ci-après.

Le monomère A est le produit « Ethacure 300 » (fournisseur Albemarle, Belgique), disponible sous forme d'un liquide relativement visqueux, de couleur brunâtre ; il est constitué à environ 96% d'un mélange d'isomères 3,5-bis(méthylthio)-2,4-toluènediamine et de 3,5-bis(méthylthio)2,6-toluènediamine (rapport pondéral d'environ 4/1 d'après analyse chromatographique).

Le monomère B est le 1,4-cyclohexane diméthanol diglycidyl éther (en abrégé « CHXDE ») (fournisseur Aldrich, Suisse), disponible sous forme d'un grade technique (environ 61% d'un mélange de diépoxydes cis/trans). Selon les données techniques disponibles (par exemple demande de brevet US 2011/0039982), le CHXDE est un mélange chimique complexe contenant les diépoxydes (env. 61%), du cyclohexane diméthanol résiduel (environ 2%), des monoépoxydes (environ 8%) et enfin des espèces époxydes polymériques (environ 29%). D'après l'analyse RMN, la masse équivalente d'époxyde « EEW » (*epoxide equivalent weight*) du composé ici utilisé est égale à environ 159 (EEW théorique égal à 128 dans le cas d'un composé pur).

Lors d'une première étape, les deux Monomères A et B ci-dessus ont tout d'abord été purifiés comme suit.

Le produit « Ethacure 300 » (environ 40 g) est déposé dans une colonne de chromatographie (700 g de silice) ; on utilise un mélange de solvant hexane/ dichlorométhane/ acétate d'éthyle (rapports 10:8:2) comme phase mobile. L'isomère 2,4-toluènediamine est élué en premier, suivi du deuxième isomère 2,6-toluènediamine. Dans ces conditions, les impuretés (couleur vert-bleu) ne sont pas éluées et peuvent être facilement séparées des constituants visés.

L'identification des isomères 2,4 et 2,6 est confirmée par l'analyse RMN¹H (500 MHz) dans le solvant DMSO-d6, qui donne les résultats suivants :
- 3,5-bis(méthylthio)-2,4-toluènediamine:
   6.96 (s, *1H), 5.21-5.23 (d, 4H),* 2.16 (s, *3H), 2.10 (s, 3H),* 1.97 (s, *3H).*
- 3,5-bis(méthylthio)-2,6-toluènediamine:
   7.22 (s, *1H), 5. 09* (s, *4H), 2.15 (s, 6H),* 1.92 (s, *3H).*

Le produit « CHXDE » est quant à lui isolé par distillation sous vide selon les conditions d'expérimentation décrites dans l'exemple 3 du document précité US 2011/0039982. Les structures cis/trans du diépoxyde sont confirmées par analyse RMN ¹H dans DMSO-d6.

Après ces étapes de purification, les Prépolymères 1 et 2 conformes à l'invention ont été préparés comme schématisé respectivement aux figures 6 et 9, selon les informations plus détaillées qui suivent.

Dans un ballon à trois cols de 25 ml préalablement séché (100°C sous vide), muni d'un barreau magnétique, d'un réfrigérant pourvu d'une circulation pour azote, on place 180 mg de Monomère B (0,702 mmol) puis 3 ml de tétrahydrofurane (THF). Puis, sous agitation, on ajoute 150 mg de Monomère A (soit 0,702 mmol). Le mélange obtenu est chauffé à reflux à 70°C durant une heure, puis le THF est éliminé par distillation. Le liquide transparent ainsi obtenu (Prépolymère 1 ou Monomère C1 conforme à l'invention) a été analysé par DSC de - 80°C à 200°C : la Tg mesurée (second passage) est égale à environ -40°C.

Le Prépolymère 2 (Monomère C2) a été préparé de manière similaire, à partir des deux mêmes composés (150 mg de Monomère A et 180 mg de Monomère B), le mélange étant chauffé à 170°C durant une heure et demie (sans solvant THF) dans un ballon de 25 ml muni d'un réfrigérant, la réaction s'effectuant à l'air. Le produit de réaction ainsi obtenu (Prépolymère 2 ou Monomère C2 conforme à l'invention) présentait une Tg d'environ + 31°C (second passage de DSC).

### 5.2. Essai 2 - Synthèse des Polyurées 1 et 2

Puis, dans une étape finale telle que schématisée respectivement aux figures 7 et 10, les Polyurées 1 et 2 ont été synthétisées par réaction des Prépolymères 1 et 2 selon l'invention avec le Monomère D (MDI bloqué caprolactame), comme décrit en détail ci-après.

Dans un récipient en verre, on place 330,5 mg de Monomère C1 (Prépolymère 1) puis 336 mg de Monomère D (« Grilbond IL6 ») dans 8 ml de solvant DTP (1,3-diméthyl-3,4,5,6-tétrahydro-2(*1H*-pyrimidinone; CAS 7226-23-5). La suspension est placée quelques secondes sous vibration mécanique (dispositif vortex) jusqu'à la dissolution complète du Monomère D.

Puis, on dépose de manière uniforme, 1,5 ml de cette solution sur une plaque en verre (10x10 cm) ; la plaque de verre est ensuite placée dans une étuve (ventilation à air) à 170°C durant 15 min, étape suivie de 15 min à 190°C sous vide afin d'éliminer les traces de solvant.

La couche mince de Polyurée 1 ainsi obtenue a révélé une excellente adhésion au verre (impossibilité de séparer par traction le polymère du verre). Cette Polyurée P1, analysée par DSC, présentait une Tg d'environ 190°C (second passage).

Pour la synthèse de la Polyurée 2, le Prépolymère 2 (Monomère C2, 0,702 mmol) a été dissous partiellement dans 12 ml de DTP chauffé dans un premier temps pendant 2 min à environ 110°C. Puis, en chauffant le milieu à 170°C durant 30 min, on obtient un produit bien soluble. On ajoute ensuite 336 mg de Monomère D (0,702 mmol) et mélangé le tout dans un agitateur (dispositif vortex) jusqu'à dissolution complète du Monomère D.

Puis on a déposé, de manière uniforme, 4 ml de la solution ainsi obtenue sur une plaque en acier zingué (surface 100 cm²); le tout a été placé dans une étuve (ventilation à l'air) à 170°C durant 15 min. Le film transparent jaune a été ensuite traité sous vide à 190°C pendant 15 min afin d'éliminer les traces de solvant.

La Polyurée 2 finale ainsi obtenue, sous la forme d'un film fin, a révélé une excellente adhésion à la plaque en acier (impossibilité de séparer par traction le polymère du métal). Cette Polyurée 2, analysée par DSC entre -80°C et +200°C (selon une rampe de 10°C/min) présentait une Tg d'environ 160°C (second passage).

### 5.3. Essai 3 - Synthèse de Polyurée 3 et tests d'adhésion en composite métal / caoutchouc

Lors d'un essai supplémentaire, on a synthétisé un nouveau polymère (Polyurée 3) par réaction en solution (solvant DTP) du Monomère C1 conforme à l'invention (Prépolymère 1, produit d'addition des monomères A et B tous deux utilisés dans cet essai sous forme purifiée) avec du MDI solide (ici sous forme non bloqué, Sigma Aldrich), selon le mode opératoire qui suit.

Dans un appareillage sec équipé d'un ballon à trois cols de 250 ml, d'un réfrigérant, d'un barreau magnétique et d'une sortie d'azote, sont ajoutés 1,5 g de Monomère B purifié (4^{ème} fraction) puis 20 ml de solvant DTP. Dans un même temps, on a préparé une première solution de 1,5 g de 3,5-bis(méthylthio)-2,4-toluènediamine (isolée du produit « Ethacure 300 ») dans 30 ml de DTP qui a été placée dans une ampoule à addition et une seconde solution de 1,75 g de diphénylméthane diisocyanate (MDI) dissout dans 30 ml de DTP et placée dans une seconde ampoule à addition, le tout sous azote.

Sous agitation et à 100°C (température intérieure du ballon), on a ajouté goutte à goutte les solutions d'amine aromatique et de MDI. La réaction de polymérisation a été poursuivie durant 4 h à 100°C, suivie d'une étape de 1 h à 160°C.

La Polyurée 3 ainsi obtenue, sous forme d'une solution transparente jaune, a été analysée par DSC entre -80°C et +200°C (selon une rampe de 10°C/min) ; elle présentait une Tg égale à environ 150°C (second passage).

Puis cette solution de Polyurée 3 (0,1 ml) a été déposée sur un feuillard en acier laitonné (acier avec taux de carbone égal à 1%) de dimensions 10 cm x 0,5 cm x 0,2 mm ; puis le tout a été placé à l'étuve à 175°C (ventilation à l'air) durant 15 min, puis 15 min supplémentaires à 175°C sous vide afin d'éliminer toute trace de solvant.

Le feuillard a été ensuite refroidi à température ambiante puis on a déposé, sur la fine couche de film polyurée ainsi formée, 0,75 ml d'une solution de caoutchouc naturel époxydé (ENR, avec un taux d'époxydation de 25% ; fournisseur Aldrich) et de diisocyanate (MDI liquide « Suprasec » 2020 de Huntsman) qui a été préparée comme suit : 150 mg de ENR ont été dissous préalablement dans 5 ml de toluène, pendant 30 min à 23°C sous agitation (barreau magnétique) ; on a ensuite ajouté, sous courant d'azote, 100 mg de diisocyanate. Le feuillard ainsi préparé a été finalement traitée à l'étuve à 100°C sous vide, pendant 15 min.

Le feuillard en acier laitonné ainsi revêtue du film de Polyurée 3 elle-même recouverte de la fine couche de ENR a été ensuite placé dans une composition de caoutchouc conventionnelle pour armature de ceinture de pneu tourisme, à base de caoutchouc naturel, de noir de carbone et silice à titre de charge, et d'un système de vulcanisation (soufre et accélérateur sulfénamide) ; cette composition étant dépourvue de sel de cobalt.

Puis l'éprouvette de composite métal/caoutchouc ainsi préparée a été placée sous presse et le tout cuit (vulcanisé) à 165°C pendant 30 min sous une pression de 20 bar.

Après vulcanisation du caoutchouc, on a obtenu un excellent collage entre la matrice de caoutchouc et le feuillard en acier laitonné malgré l'absence de sel de cobalt dans la matrice de caoutchouc : lors de tests de pelage conduits à la fois à température ambiante (23°C) et à haute température (100°C), on a constaté en effet que la rupture se produisait systématiquement dans la matrice de caoutchouc elle-même et non à l'interphase entre métal et caoutchouc.

En conclusion, les polyamines polyaromatiques soufrées de l'invention permettent de synthétiser des polyurées qui se caractérisent par une température de transition vitreuse élevée, une haute stabilité thermique et chimique, et une excellente adhésion au verre ou au métal.

Grâce à l'invention, ces polyurées, utilisées comme primaire d'adhésion sur métal dans des composites métal / caoutchouc, permettent très avantageusement de coller ensuite le métal aux matrices de caoutchouc en utilisant de simples colles textiles telles que des colles « RFL » (résorcinol-formaldéhyde-latex) ou autres compositions adhésives équivalentes, ou encore directement (c'est-à-dire sans emploi de telles colles) à ces matrices de caoutchouc lorsque ces dernières contiennent par exemple des élastomères insaturés fonctionnalisés appropriés tels que des élastomères époxydés.

Ainsi peuvent être supprimés notamment les sels de cobalt (ou autres sels métalliques) dans les compositions de caoutchouc destinées à être reliées à des renforts métalliques laitonnés.

## Revendications

1. Composé polyamine polyaromatique soufrée répondant à la formule (I) :
H₂N-Ar₁-NH-CH₂-CH(OH)-(CH₂)ₘ-O-Z₁-O-(CH₂)ₘ-CH(OH)-CH₂-[X]ₙ-HN-Ar₂-NH₂
dans laquelle X représente l'enchaînement :
-HN-Ar₃-NH-(CH₂)-CH(OH)-(CH₂)ₘ-O-Z₂-O-(CH₂)ₘ-CH(OH)-(CH₂)-
et dans laquelle:
- le symbole « n » représente un nombre entier égal à zéro ou différent de zéro ;
- les symboles « m », identiques ou différents, représentent un nombre entier compris dans un domaine de 1 à 10, de préférence de 1 à 5 ;
- les symboles Z₁ et Z₂, identiques ou différents, représentent un groupement de liaison divalent comportant de 1 à 30 atomes de carbone et pouvant comporter un hétéroatome ;
- Ar₁, Ar₂ et Ar₃, identiques ou différents, représentent un groupe phénylène, un au moins de ces groupes phénylène étant porteur de un, deux, trois ou quatre groupements de formule -Sₓ-R dans laquelle « x » est un entier de 1 à 8 et R représente l'hydrogène ou un groupe hydrocarboné, pouvant comporter un hétéroatome, comportant de 1 à 10 atomes de carbone.

2. Composé selon la revendication 1, dans lequel « n » est égal à 1.

3. Composé selon la revendication 1, dans lequel « n » est compris dans un domaine de 2 à 20, de préférence de 2 à 10.

4. Composé selon l'une quelconque des revendications 1 à 3, dans lequel les symboles « m », identiques ou différents, représentent un nombre entier égal à 1 ou 2, de préférence égal à 1.

5. Composé selon l'une quelconque des revendications 1 à 4, dans lequel Z₁ et Z₂, identiques ou différents, représentent un groupement aliphatique comportant de 2 à 20 atomes de carbone ou cycloaliphatique comportant de 3 à 20 atomes de carbone.

6. Composé selon la revendication 5, dans lequel Z₁ et Z₂ représentent le cyclohexane 1,4-diméthylène.

7. Composé selon l'une quelconque des revendications 1 à 6, dans lequel chacun des groupes Ar₁, Ar₂ et Ar₃, est porteur de 1, 2, 3 ou 4 groupements de formule -Sₓ-R.

8. Composé selon l'une quelconque des revendications 1 à 7, dans lequel « x » est compris dans un domaine de 1 à 4, de préférence égal à 1 ou 2.

9. Composé selon l'une quelconque des revendications 1 à 8, dans lequel R est un alkyle ayant de préférence de 1 à 5 atomes de carbone.

10. Composé selon la revendication 9, dans lequel R représente un méthyle ou un éthyle, de préférence un méthyle.

11. Composé selon l'une quelconque des revendications 1 à 10, dans lequel les enchaînements phénylènediamino monovalents H₂N-Ar₁-NH- et -HN-Ar₂-NH₂, identiques ou différents, répondent à l'une des formules (III-a), (III-b) ou (III-c) :

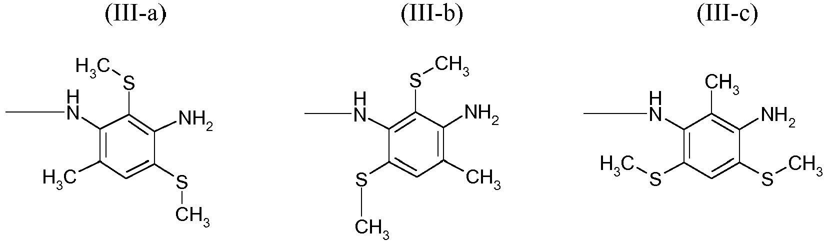

12. Composé selon l'une quelconque des revendications 1 à 11, dans lequel les enchaînements phénylènediamino divalents -HN-Ar₃-NH-, identiques ou différents, répondent à l'une des formules (III-d) ou (III-e) :

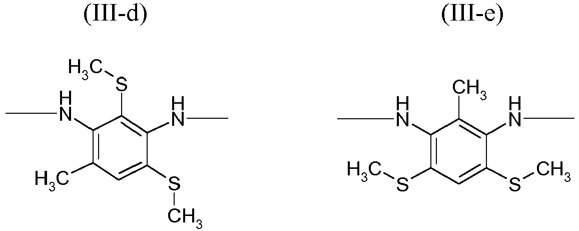

13. Polyurée issue d'au moins un composé polyamine polyaromatique soufrée selon l'une quelconque des revendications 1 à 12.

14. Procédé de synthèse d'une polyurée, par polycondensation d'au moins un composé polyamine polyaromatique soufrée selon l'une quelconque des revendications 1 à 12, avec un composé polyisocyanate.

15. Utilisation d'un composé polyamine polyaromatique soufrée selon l'une quelconque des revendications 1 à 12, pour la fabrication d'une polyurée.

## Patentansprüche

1. Schwefelhaltige polyaromatische Polyaminverbindung der Formel (I):
H₂N-Ar₁-NH-CH₂-CH (OH) - (CH₂)ₘ-O-Z₁-O- (CH₂)ₘ-CH(OH) -CH₂-[X]ₙ-HN-Ar₂-NH₂
wobei X für die Kette:
-HN-Ar₃-NH- (CH₂) -CH(OH) - (CH₂)ₘ-O-Z₂-O-(CH₂)ₘ-CH (OH) - (CH₂)-steht und wobei:
- das Symbol "n" für eine ganze Zahl, die gleich null oder von null verschieden ist, steht;
- die Symbole "m", die gleich oder verschieden sind, für eine ganze Zahl in einem Bereich von 1 bis 10, vorzugsweise 1 bis 5, stehen;
- die Symbole Z₁ und Z₂, die gleich oder verschieden sind, für eine zweiwertige Verknüpfungsgruppe, die 1 bis 30 Kohlenstoffatome enthält und ein Heteroatom enthalten kann, stehen;
- Ar₁, Ar₂ und Ar₃, die gleich oder verschieden sind, für eine Phenylengruppe stehen, wobei mindestens eine dieser Phenylengruppen eine, zwei, drei oder vier Gruppen der Formel -Sₓ-R trägt, wobei "x" eine ganze Zahl von 1 bis 8 ist und R für Wasserstoff oder eine Kohlenwasserstoffgruppe, die ein Heteroatom enthalten kann und 1 bis 10 Kohlenstoffatome enthält, steht.

2. Verbindung nach Anspruch 1, wobei "n" gleich 1 ist.

3. Verbindung nach Anspruch 1, wobei "n" in einem Bereich von 2 bis 20, vorzugsweise 2 bis 10, liegt.

4. Verbindung nach einem der Ansprüche 1 bis 3, wobei die Symbole "m", die gleich oder verschieden sind, für eine ganze Zahl mit einem Wert von 1 oder 2, vorzugsweise 1, stehen.

5. Verbindung nach einem der Ansprüche 1 bis 4, wobei Z₁ und Z₂, die gleich oder verschieden sind, für eine aliphatische Gruppe mit 2 bis 20 Kohlenstoffatomen oder eine cycloaliphatische Gruppe mit 3 bis 20 Kohlenstoffatomen stehen.

6. Verbindung nach Anspruch 5, wobei Z₁ und Z₂ für Cyclohexan-1,4-dimethylen stehen.

7. Verbindung nach einem der Ansprüche 1 bis 6, wobei jede der Gruppen Ar₁, Ar₂ und Ar₃ 1, 2, 3 oder 4 Gruppen der Formel -Sₓ-R trägt.

8. Verbindung nach einem der Ansprüche 1 bis 7, wobei "x" in einem Bereich von 1 bis 4 liegt und vorzugsweise gleich 1 oder 2 ist.

9. Verbindung nach einem der Ansprüche 1 bis 8, wobei R ein Alkyl mit vorzugsweise 1 bis 5 Kohlenstoffatomen ist.

10. Verbindung nach Anspruch 9, wobei R für ein Methyl oder ein Ethyl, vorzugsweise ein Methyl, steht.

11. Verbindung nach einem der Ansprüche 1 bis 10, wobei die einwertigen Phenylendiamino-Ketten H₂N-Ar₁-NH- und -HN-Ar₂-NH₂, die gleich oder verschieden sind, einer der Formeln (III-a), (III-b) oder (III-c) entsprechen:

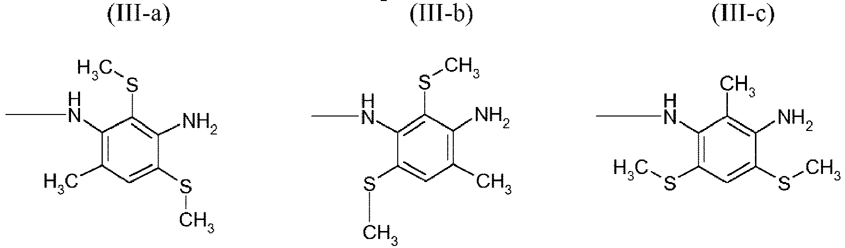

12. Verbindung nach einem der Ansprüche 1 bis 11, wobei die zweiwertigen Phenylendiamino-Ketten -HN-Ar₃-NH-, die gleich oder verschieden sind, einer der Formeln (III-d) oder (III-e) entsprechen:

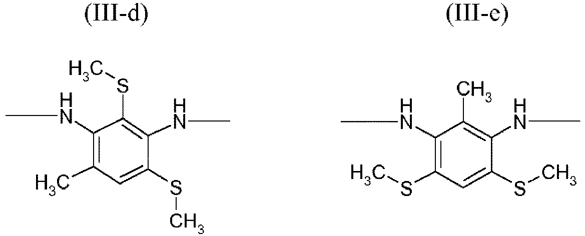

13. Polyharnstoff, erhalten aus mindestens einer schwefelhaltigen polyaromatischen Polyaminverbindung nach einem der Ansprüche 1 bis 12.

14. Verfahren zur Synthese eines Polyharnstoffs durch Polykondensation mindestens einer schwefelhaltigen polyaromatischen Polyaminverbindung nach einem der Ansprüche 1 bis 12 mit einer Polyisocyanatverbindung.

15. Verwendung einer schwefelhaltigen polyaromatischen Polyaminverbindung nach einem der Ansprüche 1 bis 12 zur Herstellung eines Polyharnstoffs.

## Claims

1. Sulphur-comprising polyaromatic polyamine compound corresponding to the formula (I):
H₂N-Ar₁-NH-CH₂-CH(OH)-(CH₂)ₘ-O-Z₁-O-(CH₂)ₘ-CH(OH)-CH₂-[X]ₙ-HN-Ar₂-NH₂
in which X represents the string:
-HN-Ar₃-NH-(CH₂)-CH(OH)-(CH₂)ₘ-O-Z₂-O-(CH₂)ₘ-CH(OH)-(CH₂)-
and in which:
- the "n" symbol represents an integer equal to zero or different from zero;
- the "m" symbols, which are identical or different, represent an integer within a range from 1 to 10;
- the Z₁ and Z₂ symbols, which are identical or different, represent a divalent bonding group comprising from 1 to 30 carbon atoms;
- Ar₁, Ar₂ and Ar₃, which are identical or different, represent a phenylene group, one at least of these phenylene groups bearing one, two, three or four groups of formula -Sₓ-R in which "x" is an integer from 1 to 8 and R represents hydrogen or a hydrocarbon group which can comprise a heteroatom and which comprises from 1 to 10 carbon atoms.

2. Compound according to Claim 1, in which "n" is equal to 1.

3. Compound according to Claim 1, in which "n" is within a range from 2 to 20 and preferably from 2 to 10.

4. Compound according to any one of Claims 1 to 3, in which the "m" symbols, which are identical or different, represent an integer equal to 1 or 2, preferably equal to 1.

5. Compound according to any one of Claims 1 to 4, in which Z₁ and Z₂, which are identical or different, represent an aliphatic group comprising from 2 to 20 carbon atoms or a cycloaliphatic group comprising from 3 to 20 carbon atoms.

6. Compound according to Claim 5, in which Z₁ and Z₂ represent cyclohexane-1,4-dimethylene.

7. Compound according to any one of Claims 1 to 6, in which each of the Ar₁, Ar₂ and Ar₃ groups bears one, two, three or four groups of formula -Sₓ-R.

8. Compound according to any one of Claims 1 to 7, in which "x" is within a range from 1 to 4, preferably is equal to 1 or 2.

9. Compound according to any one of Claims 1 to 8, in which R is an alkyl preferably having from 1 to 5 carbon atoms.

10. Compound according to Claim 9, in which R represents a methyl or an ethyl, preferably a methyl.

11. Compound according to any one of Claims 1 to 10, in which the monovalent phenylenediamino strings H₂N-Ar₁-NH- and -HN-Ar₂-NH₂, which are identical or different, correspond to one of the formulae (III-a), (III-b) and (III-c):

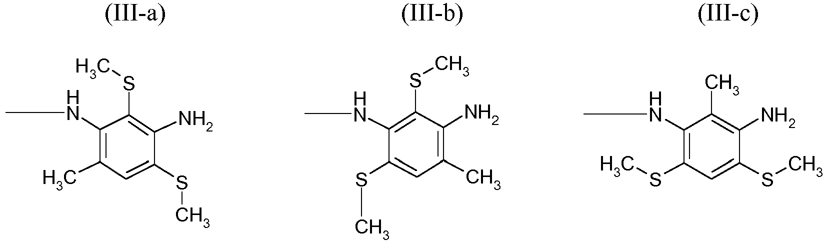

12. Compound according to any one of Claims 1 to 11, in which the divalent phenylenediamino strings -HN-Ar₃-NH-, which are identical or different, correspond to either of the formulae (III-d) and (III-e):

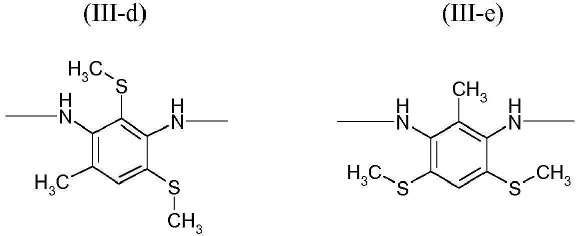

13. Polyurea resulting from at least one sulphur-comprising polyaromatic polyamine compound according to any one of Claims 1 to 12.

14. Process for the synthesis of a polyurea by polycondensation of at least one sulphur-comprising polyaromatic polyamine compound according to any one of Claims 1 to 12 with a polyisocyanate compound.

15. Use of a sulphur-comprising polyaromatic polyamine compound according to any one of Claims 1 to 12 in the manufacture of a polyurea.
